# EUROPEAN PATENT SPECIFICATION

(11) **EP 4 531 687 B1**
(45) Date of publication and mention of the grant of the patent: **18.02.2026**
(21) Application number: 23728781.8
(22) Date of filing: 29.05.2023
(51) Int. Cl.: A61B 6/00, G06T 7/00, G16H 40/60, G16H 40/40, G16H 30/40, G16H 30/20, G16H 40/63

(54) **IMPROVING THE IMAGE QUALITY OF MEDICAL IMAGES**
VERBESSERUNG DER BILDQUALITÄT MEDIZINISCHER BILDER
AMÉLIORATION DE LA QUALITÉ D'IMAGE D'IMAGES MÉDICALES

(30) Priority: 02.06.2022 EP 22176997
(43) Date of publication of application: 09.04.2025
(73) Proprietor: Koninklijke Philips N.V., 5656 AG Eindhoven (NL)
(72) Inventor: KOTHAWALA, Aliarshad Aameer, 5656 AG Eindhoven (NL); SETH, Subhendu, 5656 AG Eindhoven (NL); KAUSHAL, Nitesh, 5656 AG Eindhoven (NL); BADRINARAYAN SHARMA, Swetha, 5656 AG Eindhoven (NL); BERA, Deep, 5656 AG Eindhoven (NL); ASWATHA, Shashaank Mattur, 5656 AG Eindhoven (NL); VAJINEPALLI, Pallavi, 5656 AG Eindhoven (NL)
(74) Representative: Philips Intellectual Property & Standards
(86) International application number: PCT/EP2023/064308
(87) International publication number: WO 2023/232731

(56) References cited:
- US-A1- 2022 130 520
- JIAYU DUAN ET AL: "Image quality guided iterative reconstruction for low-dose CT based on CT image statistics", PHYSICS IN MEDICINE AND BIOLOGY, INSTITUTE OF PHYSICS PUBLISHING, BRISTOL GB, vol. 66, no. 18, 16 September 2021 (2021-09-16), pages 185018, XP020369013, ISSN: 0031-9155, [retrieved on 20210916], DOI: 10.1088/1361-6560/AC1B1B

## Description

### FIELD OF THE INVENTION

The present invention relates to the field of medical imaging, and in particular, to the field of improving image quality.

### BACKGROUND OF THE INVENTION

In medical imaging, such as ultrasound imaging, raw or partially-processed medical imaging data is processed using one or more imaging parameters to produce processed medical imaging data (e.g., medical imaging data suitable for rendering and display).

However, there is a high operator dependency involved in the adjustment and selection of such imaging parameters (with an aim to improve image quality). Thus, experienced sonographers tend to achieve high quality imaging data that accurately depicts the underlying anatomical structures of interest, whilst novice/inexperienced ultrasound operators struggle to achieve the same results.

To overcome this issue, automated workflows, in which one or more imaging parameters are automatically modified using one or more image quality metrics, are gaining importance.

It is important for the image quality metric(s) used in automated workflows to be objective, to facilitate scaling-up and standardization of workflow. Generally, objective image quality metrics may be classified into full reference image quality metrics (FR-IQMs), i.e. derived using reference imaging data, and no-reference image quality metrics (NR-IQMs), i.e. derived without using reference imaging data.

Although full reference image quality metrics demonstrate high correlation with a subjective judgement, there is typically a complete absence of the necessary reference images in clinical environments (as the location, size and shape of target objects under investigation is unknown). This limits its adaptability. Accordingly, no-reference image quality metrics are used as the next choice. However, no-reference metrics do not always correlate well with the perceived ultrasound B-mode image quality.

It would therefore be desirable to provide an improved mechanism for automated improvement to the imaging parameter(s) used to produce the medical imaging data.

Document US 2022/130520 A1 discloses a computer-implemented method for generating a patient-specific imaging protocol using a neural network having been trained to generate a probabilistic quality representation corresponding to a region of a generated simulated image. The method comprises receiving scout scan data, the received scout scan data including scout scan information and scout scan parameters, generating the generated simulated image based on the received scout scan data, scan acquisition parameters and image reconstruction parameters, deriving a simulated dose map from the received scout scan data and the scan acquisition parameters, evaluating a determined image quality of the generated simulated image relative to a predetermined image quality threshold and the derived simulated dose map relative to a predetermined dosage threshold, and generating, based on the evaluating, imaging protocol parameters based on the scan acquisition parameters and the image reconstruction parameters.

### SUMMARY OF THE INVENTION

The invention is defined by the claims.

According to examples in accordance with an aspect of the invention, there is provided a computer-implemented method of modifying a set of one or more imaging parameters for a medical imaging process.

The computer-implemented method comprises iteratively performing, until one or more first predetermined criteria are met, a first process.

The first process comprises: performing, until one or more second predetermined criteria are met, a second process comprising: obtaining raw or partially-processed medical imaging data; processing the raw or partially-processed medical imaging data using the set of one or more imaging parameters to produce processed medical imaging data; determining one or more first image quality metrics by processing the processed medical imaging data using at least one no-reference image quality metric technique; and modifying the set of one or more imaging parameters responsive to the determined one or more first image quality metrics.

The first process also comprises obtaining raw or partially-processed reference medical imaging data used for at least one full-reference image quality metric technique; processing the raw or partially-processed reference medical imaging data using the set of one or more imaging parameters to produce comparison medical imaging data; determining one or more second image quality metrics by processing the comparison medical imaging data using at least one full-reference image quality metric technique; and modifying the set of one or more imaging parameters responsive to the determined one or more second image quality metrics. As is known by a skilled person, reference imaging data is used in (or in other words, necessary for) full-reference imaging quality metric techniques. Reference imaging data used for full-reference image quality metirc technique typically are (i.e. representing) one or more targets having a known material composition, shape, position and/or size. Reference imaging data can thus be, for example, imaging data of a calibrated phantom having known targets for imaging.

The present invention proposes a bi-level or two-level approach for improving or modifying one or more imaging parameters used to process raw or partially-processed medical imaging data. A first loop or iterative process uses a no-reference image quality metric (NR-IQM) technique to produce one or more first image quality metrics, which can be used in an optimization or modification process on the imaging parameter(s). A second loop or iterative process, which includes the first loop, uses a full-reference image quality metric (FR-IQM) technique to modify the imaging parameters.

Thus, both no-reference imaging quality metrics and full-reference imaging quality metrics are used to produce the imaging parameters.

The first loop is performed using (desired) medical imaging data processed using the imaging parameter(s). Thus, the first loop can represent an optimization in the clinical domain, e.g., using clinically useful or relevant imaging data. The second loop is performed using reference medical imaging data of a calibrated phantom. A calibrated phantom is an object used for calibrating or testing medical imaging equipment, where the dimensions, size, position, acoustic and mechanical properties of objects is known. Calibrated phantoms are well known in the medical imaging field.

The present invention provides a mechanism for bridging the gap between these two worlds of IQMs - by exploiting the adherence of FR-IQMs to acoustic and objective ways of estimating image quality, yet with the flexibility and applicability of the NR-IQMs for providing the necessary clinical context for producing high-quality medical imaging data.

In the context of the present disclosure, processed medical imaging data is medical imaging data that is suitable for rendering for display at a user interface or screen. Thus, processed medical imaging data may comprise an image depicting a spatially accurate representation of the region/area imaged to obtain the raw medical imaging data. For instance, the processed medical imaging data may be reconstructed medical imaging data.

The raw or partially-processed medical imaging data is different to the raw or partially-processed reference medical imaging data. In particular, the raw or partially-processed medical imaging data may be raw or partially-processed reference medical imaging data generated "in the field", i.e., of a subject/individual/patient undergoing medical imaging.

The computer-implemented method may further comprise, after completing the first process, outputting medical imaging data produced by processing the raw or partially-processed medical imaging data using the set of one or more imaging parameters.

In some examples, the step of outputting medical imaging data comprises controlling a user interface to provide a visual representation of the medical imaging data produced by processing the raw or partially-processed medical imaging data using the set of one or more imaging parameters. This approach provides an operator or individual with high-quality medical imaging data that has been produced using an improved or optimized set of one or more imaging parameters.

The set of one or more imaging parameters may include at least gain and dynamic range.

The step of determining one or more second image quality metrics may comprise: obtaining a first property indicator that indicates one or more desired properties of medical imaging data produced by processing the raw or partially-processed medical imaging data using the set of one or more imaging parameters; selecting, from a plurality of potential full-reference image quality metric techniques, one or more full-reference image quality metric techniques responsive to the property indicator; and generating the one or more second image quality metrics by processing the comparison medical imaging data using the selected one or more full-reference image quality metric techniques.

The step of modifying the set of one or more imaging parameters responsive to the determined one or more second image quality metrics may comprise: obtaining a second property indicator that indicates one or more desired properties of medical imaging data produced by processing the raw or partially-processed medical imaging data using the set of one or more imaging parameters; generating a combined image quality metric by weighting each second image quality metric and combining the weighted second image quality metrics; and modifying the set of one or more imaging parameters responsive to the determined combined image quality metric, wherein the weighting of each second image quality metric is responsive to the property indicator.

These approaches recognize that the type of FR-IQMs used to modify the imaging parameter(s) will affect the properties of medical imaging data produced using such imaging parameter(s). By modifying which FR-IQMs are generated, or to what extent each generated FR-IQM is used in the modification of the set of one or more imaging parameters, the imaging parameter(s) can be guided or controlled towards those that produce the most suited or desired properties of medical imaging data produced using such imaging parameters.

In some examples, the step of modifying the set of one or more imaging parameters responsive to the determined one or more first image quality metrics comprises processing, using a first optimization algorithm, the one or more first image quality metrics and the set of imaging parameters to thereby obtain an improved set of one or more imaging parameters.

Optionally, the step of modifying the set of one or more imaging parameters responsive to the determined one or more second image quality metrics comprises processing, using a second optimization algorithm, the one or more second image quality metrics and the set of imaging parameters to thereby obtain an improved set of one or more imaging parameters.

The first and/or second optimization algorithm (where present) may be a heuristic optimization algorithm.

Heuristic optimization algorithms are not concerned with finding the global maximum/maxima of the objective function and, instead, may find a/the local maximum/maxima which approximates the global maximum/maxima. This significantly reduces the processing resources needed for the optimization algorithm and enables the optimization algorithm to output an optimal set of parameters quicker. Additionally, the quicker processing times may further enable the optimization algorithm to be used in real time.

The heuristic optimization algorithm may be one of an iterative optimization algorithm and a gradient descent optimization algorithm.

In some examples, the obtained raw or partially processed medical imaging data is the same for each iteration of the second process. This maintains the necessary context of the raw or partially processed medical imaging data to ensure consistent optimization of the imaging parameter(s).

In some examples, the step of determining one or more second image quality metrics comprises: obtaining calibration medical imaging data, being processed imaging data representing a known view, material composition, shape, precisely estimated acoustic and mechanical properties, position and/or size of each target; and processing the comparison medical imaging data and the calibration medical imaging data to produce the one or more second image quality metrics.

In some examples, the step of obtaining raw or partially-processed reference medical imaging data comprises, for at least one iteration of the first process: identifying a plurality of instances of potential raw or partially-processed reference medical imaging data; and selecting one of the plurality of instances of potential raw or partially-processed reference medical imaging data as the obtained raw or partially-processed reference medical imaging data responsive to one or more characteristics of the raw or partially processed medical imaging data.

The one or more characteristics of the raw or partially processed medical imaging data may be a type of the medical imaging data, e.g., an identifier of the imaging modality (e.g., identifying whether the medical imaging data comprise an ultrasound image, a MR image, a CT image a PET image and so on). Another suitable example of a characteristic is, for partially processed medical imaging data, a processing progress (e.g., location in a processing pipeline or workflow) of the partially processed medical imaging data.

The one or more characteristics can be provided via a user input provided at a user interface, e.g., to allow an operator to specify the one or more characteristics. Other approaches for obtaining the one or more characteristics include: automatic determination based on the current clinical application; automatic retrieval from another database or data system (e.g. a patient record that specifies which type of medical imaging/exam has taken place); and/or determined by an automated classification of the raw or partially processed medical imaging data, e.g., using AI technology.

The raw or partially processed medical imaging data may comprise raw or partially processed ultrasound imaging data.

The raw or partially processed medical imaging data may comprise raw or partially processed medical imaging data obtained in a clinical environment.

The raw or partially processed medical imaging data and the raw or partially-processed reference medical imaging data may comprise medical imaging data at a same location in an image processing pipeline for medical imaging data.

The one or more first predetermined criteria may comprise: the first process being iteratively performed no less than a first predetermined number of iterations; the one or more second image quality metrics changing by less than a first predetermined percentage or amount over a preceding second predetermined number of iterations; and/or a first override indicator being provided.

The one or more second predetermined criteria may comprise: the second process being iteratively performed no less than a third predetermined number of iterations; the one or more first image quality metrics changing by less than a second predetermined percentage or amount over a preceding fourth predetermined number of iterations; and/or a second override indicator being provided.

There is also proposed computer program comprising code means for implementing any herein described method when said program is run on a processing system. There is also proposed a non-transitory computer-readable medium or data carrier having encoded thereon computer readable code configured to cause any herein described method to be performed when the code is run.

There is also proposed a processing system for modifying a set of one or more imaging parameters for a medical imaging process, the processing system being configured to iteratively perform, until one or more first predetermined criteria are met, a first process.

The first process comprises performing, until one or more second predetermined criteria are met, a second process comprising: obtaining raw or partially-processed medical imaging data; processing the raw or partially-processed medical imaging data using the set of one or more imaging parameters to produce processed medical imaging data; determining one or more first image quality metrics by processing the processed medical imaging data using a no-reference image quality metric technique; and modifying the set of one or more imaging parameters responsive to the determined one or more first image quality metrics.

The first process also comprises obtaining raw or partially-processed reference medical imaging data of a calibrated phantom having known targets for imaging; processing the raw or partially-processed reference medical imaging data using the set of one or more imaging parameters to produce comparison medical imaging data; determining one or more second image quality metrics by processing the comparison medical imaging data using a full-reference image quality metric technique; and modifying the set of one or more imaging parameters responsive to the determined one or more second image quality metrics.

The skilled person would be readily capable of adapting the processing system to carry out any herein described method and vice versa.

There is also proposed an imaging system comprising the processing system and a medical imaging device for capturing raw medical imaging data. The imaging system may further comprise a user output interface for providing a visual representation of medical imaging data produced using the set of one or more imaging parameters.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 conceptually depicts a mechanism proposed by the present disclosure;
Fig. 2 is a flowchart illustrating a method;
Fig. 3 illustrates a processing system according to an embodiment; and
Fig. 4 illustrates an imaging system according to an embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for generating a set of imaging parameters used to reconstruct medical imaging data from raw or partially-processed medical imaging data. In an inner loop, the imaging parameter(s) are modified using one or more first image metrics produced using a no-reference image quality metric technique on medical imaging data produced using the imaging parameter(s). In an outer loop, the imaging parameter(s) are modified using one or more second image metrics produced using a full-reference image quality metric technique on comparison medical imaging data. The comparison medical imaging data is produced by processing raw or partially-processed reference medical imaging data using the imaging parameter(s).

The proposed approach recognizes that a significant improvement in image quality of processed medical imaging data can be achieved if both NR-IQMs and FR-IQMs are used to modify the imaging parameter(s). The proposed approach makes use of FR-IQM to guide or direct the modifications to the imaging parameter(s) using NR-IQM, to thereby improve image quality. This approach ensures that contextual information for medical imaging data is retained in setting the imaging parameter(s), via the NR-IQMs, whilst also incorporating the fundamental explainability based on physics and image quality aspects and accuracy of FR-IQMs in setting the imaging parameter(s).

Approaches thereby facilitate the provision of higher quality medical imaging data.

Embodiments may be employed in any clinical environment in which medical imaging procedures take place, but are particularly advantageous in the processing of ultrasound imaging data.

In the field of medical imaging, production of medical imaging data is performed by capturing and processing raw medical imaging data using one or more imaging parameters. Examples of imaging parameters include selection or parameters of particular filters applied to the captured raw medical imaging data (e.g., QBP filters, Image and signal processing parameters of the ultrasound signal processing engine), and well as a gain, dynamic range and/or zoom level of the medical imaging apparatus during capture of the raw medical imaging data.

Further examples of imaging parameters include, edge enhancement parameters, edge connectivity parameters, parameters of isotropic and/or anisotropic filters; speckle smoothening parameters; contrast enhancement parameters; white busting and/or white control parameters; and/or gray level preserving and/or enhancement parameters. Other

Some imaging parameters may be specific to the type of medical imaging taking place. For instance, in ultrasound imaging, imaging parameters include blending weights for beamforming, contrast resolution enhancement (CRE) weights in frequency compounding and the like.

The processed medical imaging data is medical imaging data that is suitable for rendering for display at a user interface or screen. Thus, processed medical imaging data comprises imaging data depicting a spatially accurate representation of the region/area that was imaged when capturing the raw medical imaging data. In particular, processed medical imaging data may comprise one or more 2D and/or 3D medical images.

The processing of raw medical imaging data into processed medical imaging data (using a set of one or more imaging parameters) is sometimes called reconstructing or reconstruction. The processed medical imaging data can therefore be alternatively labelled reconstructed medical imaging data. Similarly, the imaging parameters could be alternatively labelled reconstruction parameters, as they are parameters used to generate reconstructed medical imaging data.

The procedure of processing of the raw medical imaging data can also labelled an image processing pipeline or an image processing workflow. It is possible to extract partially-processed medical imaging data at different points or locations ("tapping points") in the imaging processing pipeline. Thus, partially-processed medical imaging data is raw medical imaging data that has been captured and processed using a first set of one or more imaging parameters, but has yet to be processed using a second, different set of one or more imaging parameters to produce the processed medical imaging data.

Put another way, partially-processed medical imaging data is raw medical imaging data that has partly (but not completely) undergone a reconstruction process. The remainder of the reconstruction process, for processing the partially-processed medical imaging data to produce processed medical imaging data, makes use of one or more imaging/reconstruction parameters.

As one example, an image processing pipeline or reconstruction process for an ultrasound imaging system may include a channel radiofrequency (RF), a beamforming of channel RF to beamformed RF, processing step, an in-phase (I)/quadrature (Q) demodulation and interpolation step and/or signal-in-phase (SIP) processing step. The partially-processed medical imaging data may be data acquired at any of these steps.

It has been recognized that the quality of the medical imaging data produced by this process is dependent upon selection of the most appropriate imaging parameters for the clinical context and/or individual being imaged. For instance, different targets for a medical imaging process will require different imaging parameters for optimal medical images. As another example, different body compositions for a same medical imaging process (e.g., different levels of muscle or fatty tissue) will required different imaging parameters to achieve a same quality of processed medical imaging data.

The present disclosure proposes an approach for modifying one or more imaging parameters used to process raw or partially-processed medical imaging data. A two-level or bi-level approach is used, in which two iterative loops are performed to determine the one or more imaging parameters.

A wide variety of potential imaging parameters have been previously described and/or listed. Any of these imaging parameters are suited for use in the proposed mechanism.

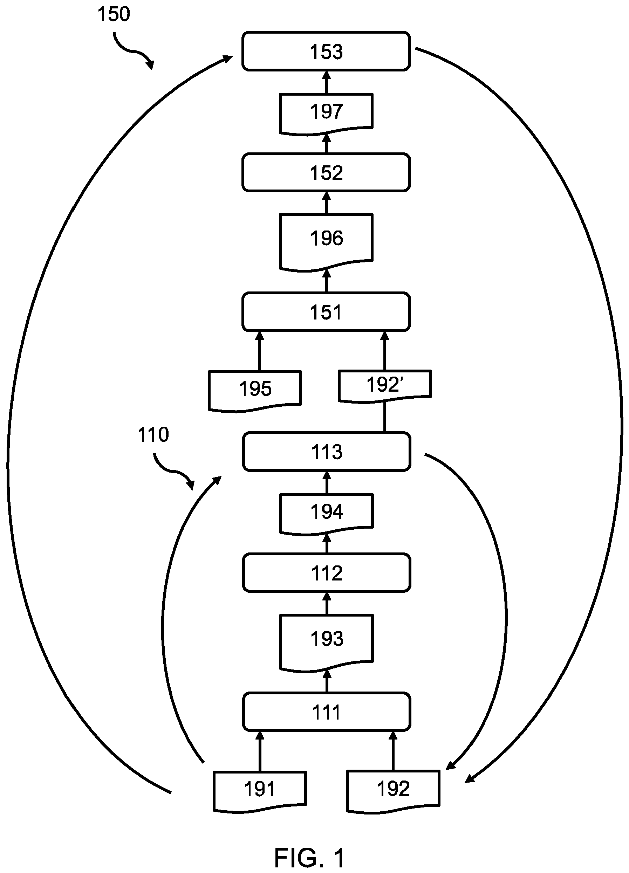

Fig. 1 conceptually illustrates the mechanism proposed in this disclosure.

The mechanism comprises an inner loop 110 and an outer loop 150. The inner loop 110 is iteratively performed within each iteration 150 of the outer loop.

The inner loop 110 iteratively processes (in a process 111) raw or partially-processed medical imaging data 191 using a set of one or more imaging parameters 192 to produce processed medical imaging data 193. The raw or partially-processed medical imaging data 191 preferably comprises raw or partially-processed medical imaging data (of raw medical imaging data) that was captured by medical imaging apparatus in a clinical environment (e.g., of a clinical subject such as a patient).

At least one first quality image metric 194 of the processed medical image is then determined in a process 112.

The process 112 makes use of one or more no-reference image quality metric (NR-IQM) determination techniques, which are well-established in the art. Examples include the BRISQUE model, the PIQE score, the Natural/Naturalness Image Quality Evaluator (NIQE) blind image quality assessment, the Neural Image (NIMA) assessment, one or more confidence scores of an AI model or neural network and so on. This approaches attempt to define image quality by adopting statistical methods combined with the Mean Opinion Scores (MOS) obtained from expert human observers.

Other approaches are known to the skilled person, and a working example will be provided later in this disclosure.

Thus, the first quality image metric(s) comprise(s) one or more no-reference image quality metrics. In particular, the first quality image metric(s) may comprise only (i.e., is or are) the one or more no-reference image quality metrics.

The at least one first quality image metric 194 is then used to modify, the set of one or more imaging parameters 192 in a first modification step 113. The first modification step can effectively act as an optimization process for deriving or producing an improved set of one or more imaging parameters 192' that improve the one or more first image quality metrics 194, thereby improving image quality.

The outer loop 150 iteratively processes (in a process 151) raw or partially-processed reference medical imaging data 195 using the same set of one or more imaging parameters 192', produced by the inner loop 110, to produce comparison medical imaging data 196. Thus, the set of one or more imaging parameters 192 modified in the inner loop 110 are re-used to process the reference medical imaging data.

The raw or partially-processed reference medical imaging data 195 is of (i.e., represents) one or more targets having a known view, material composition, shape, position, acoustic and mechanical properties, and/or size. Thus, if fully processed using an image processing pipeline, the processed reference medical imaging data would/should depict a spatially accurate representation of the one of more known targets.

The reference raw or partially-processed reference medical imaging data 195 may be data at a/the same point or location in the image processing pipeline (e.g., of a processing procedure or reconstruction) as the raw or partially-processed medical imaging data 191.

The outer loop 150 also performs a process 152 of determining at least one second quality image metric 197 of the comparison medical imaging data 196.

The process 152 makes use of one or more full-reference image quality metric (FR-IQM) determination techniques, which are well known in the art. Examples include determination of a point-spread-function (PSF), a contrast-to-noise ratio (CNR), speckle quality, and/or a signal-to-noise ratio (SNR). These image quality metrics benefit from having a physical meaning, and are therefore easy to interpret.

Thus, the second quality image metric(s) comprise(s) one or more full-reference image quality metrics. In particular, the second quality image metric(s) may comprise only (i.e., is/are) the one or more full-reference image quality metrics.

The at least one second quality image metric 197 is then used to modify, in a second modification step 153, the set of one or more imaging parameters 192'. The step 153 can effectively act as an optimization process for deriving or producing an improved set of one or more imaging parameters 192 that improve the one or more first image quality metrics 194, thereby improving image quality.

In this way, in the outer loop 150, the solution for the imaging parameter(s) derived from the inner loop is applied on pre-loaded or reference data acquired, followed by computing its FR-IQMs.

The objective of the outer loop is to effectively judge whether each inner loop is able to supply an optimized or improved solution to the next level, which in turn boosts its own improvement or optimization objective. The proposed technique thereby acts like a master and slave approach, where the improvement in image quality is directed by the more explainable and accurate FR-IQMs.

The first and second loops may each be repeated until a respective set of predetermined criteria are met. The predetermined criteria may be any suited predetermined criteria for an optimization or improvement process, which is known in the art. Suitable examples are provided later in the disclosure.

The proposed mechanism combines two forms of image quality assessment through a bi-level framework, which is able to work directly on clinically obtained raw or partially-processed medical imaging data whilst benefitting from the more accurate image quality assessment of full-reference image quality metric techniques.

The introduction of FR-IQM in quantitatively estimating the image quality of medical imaging data will bring the inherent explainability of full-reference image quality metrics into the medical imaging analysis and imaging parameter modification, covering the shortfall of no-reference image quality metrics alone. This will also help in objective optimization or improvement of the imaging parameters.

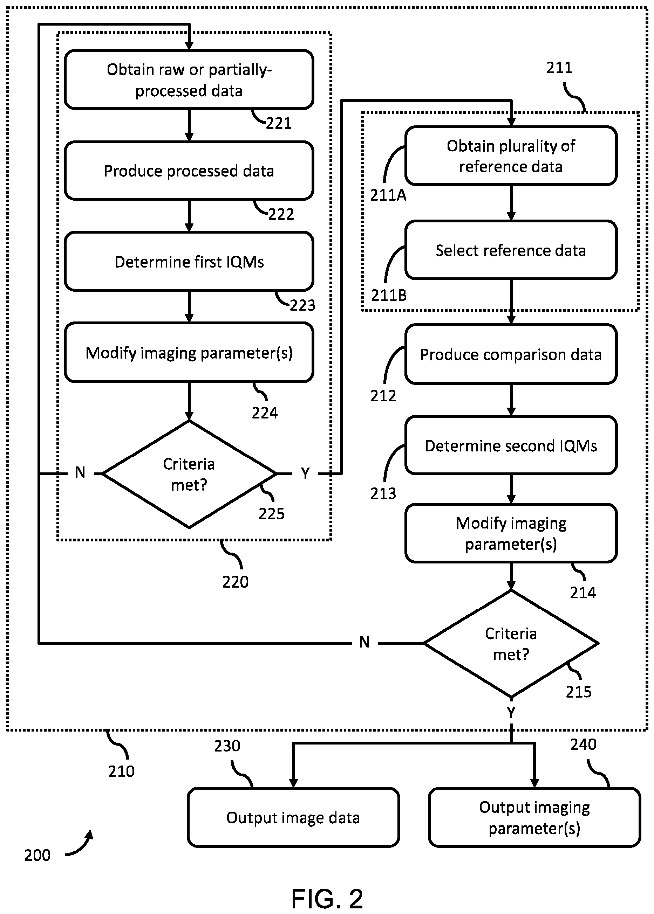

Fig. 2 illustrates a computer-implemented method 200 according to an embodiment. The computer-implemented method 200 employs the proposed mechanism to define a set of one or more parameters for a medical imaging process.

The computer-implemented method 200 comprises iteratively performing a first process 210. This first process 210 represents the outer loop previously described. The first process 210 is repeated until one or more first predetermined criteria are met.

The first process 210 first comprises iteratively performing a second process 220. The second process is itself repeated until one or more second predetermined criteria are met.

The second process 220 comprises a step 221 of obtaining raw or partially-processed medical imaging data. The raw or partially-processed medical imaging data may be, or be derived from, medical imaging data captured by a medical imaging system, such as an ultrasound system or MRI system.

The second process 220 also comprises a step 222 of processing the raw or partially-processed medical imaging data using the set of one or more imaging parameters to produce processed medical imaging data.

For a first iteration of the second process in a first iteration of the first process, the set of one or more imaging parameters may comprise or be a default set of one or more imaging parameters and/or a user-defined (e.g., via a user interface) set of one or more imaging parameters. For subsequent iterations, as will be later apparent, the set of one or more imaging parameters may be those produced during modifications made during the execution of method 200.

The second process 220 also comprises a step 223 of determining one or more first image quality metrics (IQMs) by processing the processed medical imaging data using at least one no-reference image quality metric technique. Any suitable no-reference image quality metric technique can be employed for this step, such as those previously described. Examples include BRISQUE, PIQE, NIQE, one or more confidence scores of a neural network or AI model, and/or NIMA techniques, although others will be apparent to the skilled person.

In this way, the one or more first image quality metrics comprises comprise(s) one or more no-reference image quality metrics. In particular, the first quality image metric(s) may comprise only (i.e., is or are) the one or more no-reference image quality metrics.

The second process 220 also comprises a step 224 of modifying the set of one or more imaging parameters responsive to the determined one or more first image quality metrics.

In particular, modifying the set of one or more imaging parameters may comprise modifying the one or more imaging parameters with an aim or target of improving the imaging parameter(s), e.g., such that processing the raw or partially-processed medical imaging data using the improved imaging parameter(s) would result in processed medical imaging data having more desirable one or more first image quality metrics.

Step 224 may for instance comprise processing, using a first optimization algorithm, the one or more first image quality metrics and the set of imaging parameters to thereby obtain an improved set of one or more imaging parameters.

If the one or more first image quality metrics comprises a plurality of first image quality metrics, then step 224 may comprise processing the first IQMs to produce a first combined image quality metric (IQM). The first combined IQM may act as a cost function output or evaluation metric in modifying the set of imaging parameters. Combining first IQMs may comprise performing a sum, a weighted sum, an average or a weighted average of the first IQMs or non-linear function or combination of IQMs to produce the first combined IQM.

In each iteration of the second process 220, the obtained raw or partially processed medical imaging data is preferably the same. More particularly, in each iteration of the second process 220 and each iteration of the first process 210, it is preferable for the obtained raw or partially processed medical imaging data to be the same. This maintains the necessary context of the raw or partially processed medical imaging data to ensure consistent optimization of the imaging parameter(s). However, this requirement is not essential.

The second process 220 is repeated until one or more second predetermined criteria are met, this may be determined in determination step 225.

After the second process 220 ends, the first process 210 moves to a step 211 of obtaining raw or partially-processed reference medical imaging data for one or more targets having a known view, material composition, shape, position and/or size.

The raw or partially-processed reference medical imaging data may, for instance, be raw or partially-processed reference medical imaging data of a calibrated phantom. A calibrated phantom is a physical model that contains areas, objects, elements or regions ("targets") of a known material composition, shape, well characterized acoustic and mechanical properties, position and/or size, e.g., tissue-mimicking material.

As another example, the raw or partially-processed reference medical imaging data may be synthesized raw or partially-processed reference medical imaging data of a simulated scene containing simulations of areas, objects, elements or regions ("targets") of a known or predetermined material composition, shape, position and/or size, e.g., tissue-mimicking material. Methods for synthesizing raw or partially-processed reference medical imaging data are known in the art,

As yet another example, the raw or partially-processed reference medical imaging data may be of patient or individual for whom the material composition, shape, position and/or size of one or more anatomical elements (i.e., "targets") are known.

Optionally, in at least one iteration of the first process, step 211 may comprise a sub-step 211A of identifying a plurality of instances of potential raw or partially-processed reference medical imaging data. Step 211 may also comprise a sub-step 211B of selecting one of the plurality of instances of potential raw or partially-processed reference medical imaging data as the obtained raw or partially-processed reference medical imaging data responsive to one or more characteristics of the raw or partially processed medical imaging data.

In this way, the potential raw or partially-processed reference medical imaging data may be selected, e.g., from a bank of potential instances. This allows the most appropriate or contextually similar raw or partially-processed reference medical imaging data to the raw or partially-processed medical imaging data to be identified and selected.

Each instance of potential raw or partially-processed reference medical imaging data may be associated with its own characteristics. Step 211B may comprise selecting the instance of potential raw or partially-processed reference medical imaging data having the most similar characteristics to the one or more characteristics of the raw or partially processed medical imaging data (e.g., sharing the same characteristics).

This approach improves a contextual relevance of the raw or partially-processed reference medical imaging data to the current clinical environment or context of the raw or partially-processed medical imaging data.

The one or more characteristics of the raw or partially processed medical imaging data may be a type of the medical imaging data, e.g., an identifier of the imaging modality. This may include, for instance, identifying whether the medical imaging data comprises an ultrasound image, a MR image, a CT image a PET image and so on.

Yet another suitable characteristic of the raw or partially processed medical imaging data is a view, or intended view, represented by the raw or partially processed medical imaging data. Different views would benefit from being processed according using different sets of one or more imaging parameters.

Yet another suitable characteristic of the raw or partially processed medical imaging data is a target, or intended target, of the raw or processed medical imaging data - i.e., a target, such as an anatomical feature or element, that the raw or partially processed medical imaging data represents.

Another suitable example of a characteristic is, for partially processed medical imaging data, a processing progress (e.g., location in a processing pipeline or workflow) of the partially processed medical imaging data. A processing progress effectively identifies a point in the image processing pipeline or reconstruction process from which the partially-processed reference medical imaging data is extracted.

The one or more characteristics can be provided via a user input provided at a user interface, e.g., to allow an operator to specify the one or more characteristics.

Other approaches for obtaining the one or more characteristics include: automatic determination based on the current clinical application; automatic retrieval from another database or data system (e.g. a patient record that specifies which type of medical imaging/exam has taken place); and/or determined by an automated classification of the raw or partially processed medical imaging data, e.g., using AI technology.

In preferred examples, the raw or partially processed medical imaging data and the raw or partially-processed reference medical imaging data are medical imaging data at a same location in an image processing pipeline for medical imaging data. This feature can be achieved through appropriate configuration of steps 211A and 211B.

The first process 210 then moves to a step 212 of processing the raw or partially-processed reference medical imaging data using the set of one or more imaging parameters to produce comparison medical imaging data.

The processing performed in step 212 may be identical or similar to the processing performed in step 222, but using the set of one or more imaging parameter(s) produced from the second process 220 and the raw or partially-processed reference medical imaging data obtained in step 211.

The first process 210 then moves to a step 213 of determining one or more second image quality metrics by processing the comparison medical imaging data using at least one full-reference image quality metric technique. Any suitable full-reference image quality metric technique can be employed for this step, such as those previously described. Examples include PSF, CNR, speckle quality and SNR techniques, although others will be apparent to the skilled person.

Thus, the second quality image metric(s) comprise(s) one or more full-reference image quality metrics. In particular, the second quality image metric(s) may comprise only the one or more full-reference image quality metrics.

Step 213 may be performed, for instance, by obtaining calibration medical imaging data and comparing the comparison medical imaging data to the calibration medical imaging data to produce the one or more second image quality metrics. The calibration medical imaging data is imaging data that represents a processed version of the raw or partially-processed reference medical imaging data, but one for which a shape, acoustic and mechanical properties, position and/or size of each target is known and accurately represented.

The first process 210 also comprises a step 214 of modifying the set of one or more imaging parameters responsive to the determined one or more second image quality metrics. Step 214 is analogous to step 224 used in the second process 220, but making use of a different quality metric or metrics.

Accordingly, step 214 of modifying the set of one or more imaging parameters may comprise modifying the one or more imaging parameters with an aim or target of improving the imaging parameter(s), e.g., such that processing the raw or partially-processed reference medical imaging data using the improved imaging parameter(s) would result in comparison medical imaging data having more desirable one or more first image quality metrics.

Step 214 may for instance comprise processing, using a second optimization algorithm, the one or more second image quality metrics and the set of imaging parameters to thereby obtain an improved set of one or more imaging parameters.

If the one or more second image quality metrics comprises a plurality of second image quality metrics, then step 214 may comprise processing the second IQMs to produce a second combined image quality metric (IQM). The second combined IQM may act as a cost function output or evaluation metric in modifying the set of imaging parameters. Combining second IQMs may comprise performing a sum, a weighted sum, an average or a weighted average of the second IQMs, or any other suitable weighted or unweighted non-linear function that processes these IQMs, to produce the second combined IQM.

The first process 210 is repeated until one or more first predetermined criteria are met. This may be determined in determination step 215.

The first and second process together produce a set of imaging parameters for processing raw or partially-processed medical imaging data to produce processed medical imaging data, i.e., reconstructed medical imaging data.

In some examples, the method 200 further comprises a step 230 of after completing the first process, outputting medical imaging data ("output medical imaging data") produced by processing the raw or partially-processed medical imaging data using the set of one or more imaging parameters.

Step 230 may be performed by processing the raw or partially-processed medical imaging data using the set of one or more imaging parameters (as modified by the first process, which includes the second process), and then outputting the processed medical imaging data.

Step 230 may comprise storing the output medical imaging data in a database or memory unit, e.g., for later reference and/or retrieval.

Step 230 may comprise passing the output medical imaging data to a further processing system or inputting the output medical imaging data in a further processing procedures, e.g., for analyzing the output medical imaging. Examples of further processing procedures include segmentation procedures, automated diagnostic procedures, classification procedures and so on.

Preferably, the step of outputting medical imaging data comprises controlling a user interface to provide a visual representation of the medical imaging data produced by processing the raw or partially-processed medical imaging data using the set of one or more imaging parameters. This provides an operator with useful clinical information, with improved image quality, for assessing and/or diagnosing an individual or object represented in medical imaging data.

In some examples, the method 200 further comprises a step 240 of, after completing the first process, outputting the set of one or more imaging parameters. Step 240 may comprise storing the set of one or more imaging parameters in a database or memory unit, e.g., for later reference and/or retrieval. Step 240 may comprise may comprise passing the set of one or more imaging parameters to a further processing system, e.g., a processing system configured to process future raw medical imaging data.

It has been recognized that the type of second IQMs used to modify the set of one or more imaging parameters will influence or guide the property/properties of the output medical imaging data. As previously mentioned, output medical imaging data is medical imaging data produced by processing the raw or partially-processed medical imaging data using the (modified) set of one or more imaging parameters.

For instance, if the second IQMs comprise only a contrast-to-noise ratio, then the output medical imaging data will have an improved contrast-to-noise ratio, e.g., at the expense of other properties such as resolution or speckle quality. As another example, if the second IQMs comprise only a speckle standard deviation, then the output medical imaging data will have a smoothened speckle, e.g., at the expense of other properties such as resolution or contrast.

This recognition can be taken into account by appropriately modifying step 213 and/or step 214, in order to guide the modification of the imaging parameter(s) to achieve desired properties of the output medical imaging data.

In particular, step 213 may comprise selecting which of a plurality of potential FR-IQM techniques are used to generate the second IQMs. The selection may be responsive to property indicator. The property indicator may indicate the desired property or properties of the output medical imaging data (e.g., high contrast, low speckle, high signal to noise ratio and so on). The selected FR-IQM techniques may be those that produce FR-IQMs (for the second IQMs) that represent a measure of these desired properties.

Thus, step 213 may comprise: obtaining a first property indicator that indicates one or more desired properties of medical imaging data produced by processing the raw or partially-processed medical imaging data using the set of one or more imaging parameters; selecting, from a plurality of potential full-reference image quality metric techniques, one or more full-reference image quality metric techniques responsive to the property indicator; and generating the one or more second image quality metrics by processing the comparison medical imaging data using the selected one or more full-reference image quality metric techniques.

As another example, step 214 may comprise producing a (second) combined image quality metric by selecting and/or weighting each second image quality metric determined in step 213 responsive to a/the property indicator.

**In** some examples, step 214 comprises weighting each second image quality metric responsive to the property indicator. A combined second image quality metric can then be produced by summing the weighted second image quality metrics, averaging the non-zero weighted second image quality metrics or otherwise processing the weighted second image quality metrics or using a non-linear function of these IQMS. The combined second image quality metric can act as the cost function output or evaluation metric for modifying the imaging parameter(s) in step 214.

It will be appreciated that a weighting applied to a second image quality metric may be a weighting of 0, to effectively deselect the second image quality metric from the processing performed in step 214.

In this way, the step of modifying the set of one or more imaging parameters responsive to the determined one or more second image quality metrics may comprise: obtaining a second property indicator that indicates one or more desired properties of medical imaging data produced by processing the raw or partially-processed medical imaging data using the set of one or more imaging parameters; generating a combined image quality metric by weighting each second image quality metric and combining the weighted second image quality metrics; and modifying the set of one or more imaging parameters responsive to the determined combined image quality metric, wherein the weighting of each second image quality metric is responsive to the property indicator.

A property indicator may be provided directed by a user or individual, e.g., via a user input interface. As another example, a property indicator may be provided from a memory or central control system, e.g., a patient database providing information about the individual being imaged and/or the operator of the medical imaging system.

The (first and/or second) property indicator may directly indicate a desired property of the output medical imaging data (e.g., high contrast).

Alternatively, the property indicator may identify a property of the imaged target and/or individual of the raw or partially-processed medical imaging data, i.e., the target and/or individual represented by the raw or partially-processed medical imaging data. It is recognized that different scenarios and properties of the individual will affect which properties of the output medical imaging data are most desirable.

For instance, it is preferred to have high resolution medical imaging data of an embryo/fetus during early pregnancy (due to the relatively small size of the feature/structure of the embryo/fetus) and high contrast medical imaging data during later pregnancy (e.g., to investigate the major anatomies and their separation from one another).

As another example, an amount of soft tissue or fat content of an individual can affect the desired property/properties of the medical imaging data. In particular, as a bulk of soft tissue attenuates signals (especially in ultrasound imaging), to perform imaging at deeper locations in human body, it may be preferable to prioritize contrast over resolution for high levels of soft tissue and large depths, compared to low levels or shallower depth of imaging. Thus, a level of soft tissue can control which FR-IQM techniques contribute most to the combined IQM.

Thus, it is possible to map one or more characteristics of the individual or target being imaged to desired properties of the output medical imaging data, e.g., in a look-up table or dataset. Each desired property may be mapped to corresponding weights for the second IQMs and/or choices/selections of the second IQMs, e.g., in a look-up table or dataset. The correspondence or mapping between a desired property and the weights/choices/selections may be predetermined, e.g., using a trained or experienced clinician, existing literature and/or based on (e.g., automated) analysis of calibration data.

It has previously been mentioned how different FR-IQM techniques produce image quality metrics representing different desirable properties of medical imaging data. Thus, each FR-IQM can represent a predicted adherence of medical imaging data (produced using the imaging parameter(s)) to a particular property. A number of examples of FR-IQM techniques and the associated property are hereafter described.

A contrast-to-noise ratio FR-IQM technique produces a contrast-to-noise metric that indicates a contrast level of medical imaging data produced using the imaging parameter(s). A signal-to-noise ratio FR-IQM technique produce a signal-to-noise metric, which indicates the detectability of hyperechoic regions like lesions from the background (that contains speckle), of medical imaging data produced using the imaging parameter(s). A point spread function FR-IQM technique produces a PSF metric that indicates a resolution of medical imaging data produced using the imaging parameter.

From the foregoing, it will be apparent that it is possible to modify which FR-IQMs are generated, or to what extend each FR-IQM is used to modify the set of one or more imaging parameters, using a property indicator. This allows for the set of imaging parameters to be tuned or guided to a set that achieves one or more desired properties of the output medical imaging data.

The property indicator can be provided via a user input provided at a user interface, e.g., to allow an operator to specify the property indicator. Other approaches for obtaining the property indicator include: automatic determination based on the current clinical application; and/or automatic retrieval from another database or data system (e.g. a patient record that specifies which type of medical imaging/exam has taken place).

TABLE 1 demonstrates the advantage of the proposed approach.

TABLE 1 indicates, for each of a plurality of image parameter modification techniques, the output gain and dynamic range (two examples of image parameters) produced using said technique. TABLE 1 also indicates the lateral resolution of imaging data produced using the set of imaging parameters provided using said technique. The smaller the lateral resolution, the better quality the imaging data - as smaller elements or features in the imaging data.

Techniques 1 - 3 of TABLE 1 demonstrate image parameter modification techniques that make use of only no-reference image quality metrics (which are reference in "Method") to modify the image parameters. Technique 4 ("Bi-Level") is the technique proposed in this disclosure, the "Bi-Level" technique.

For contextual understanding, techniques 1 - 4 were all configured to only modify the gain and dynamic range. Thus, the only modified image parameters for techniques 1 - 4 were a gain and dynamic range.

The data in TABLE 1 was produced using sample raw medical imaging data.

**TABLE 1**

| No. | Method | Gain (%) | Dynamic Range (dB) | Lateral Resolution (mm) |
|---|---|---|---|---|
| 1 | BRISQUE | 63 | 42 | 3.1028 |
| 2 | AI-Confidence Score | 47 | 52 | 2.2818 |
| 3 | AI-Confidence Score + BRISQUE | 44 | 53 | 2.0540 |
| 4 | Bi-Level | 47 | 60 | 1.9603 |

The present invention may make use of a first and/or second optimization algorithm to modify the one or more imaging parameters using the first/second image quality metrics respectively. Suitable optimization algorithms for this purpose will be apparent to the skilled person.

In particular examples, the first/second image quality metric(s) respectively is used as the evaluation metric, or are combined to produce the evaluation metric, in an objective function of the first/second optimization algorithm. The first/second optimization algorithm may attempt to find a maximum/minimum in the evaluation metric and the corresponding set of one or more imaging parameters. In some embodiments, the set of one or more imaging parameters corresponding to the maximum/minimum in the evaluation metric is selected as the modified set of one or more imaging parameters.

Preferably, the first and/or second optimization algorithm is a heuristic optimization algorithm (e.g. iterative optimization, gradient descent etc.). A heuristic optimization algorithm will search for a maximum of the objective function which may be a local maximum or a global maximum instead of explicitly searching for the global maximum. This allows the first and/or second optimization algorithm to find an improved set of imaging parameters with fewer processing resources and faster than non-heuristic optimization algorithms.

One example of a suitable heuristic optimization algorithm is the Particle Swarm Optimization (PSO) algorithm, such as those disclosed by Trelea, Ioan Cristian. "The particle swarm optimization algorithm: convergence analysis and parameter selection." Information processing letters 85.6 (2003): 317-325.

It has previously been explained how the first process, second process, inner loop and/or outer loop may be iteratively repeated until a respective set of predetermined criteria are met. Various predetermined criteria for ending a process for iterative modifications to imaging parameters would be apparent to the skilled person.

Example predetermined criteria include the process being iteratively performed no less than a predetermined number of iterations; the one or more (first or second, where relevant) image quality metrics changing by less than a predetermined percentage or amount over a preceding predetermined number of iterations; and/or an override indicator being provided (e.g., from a user at a user interface or by an interrupt system).

The set of predetermined criteria may be different for the first and second processes, i.e., for the outer loop and the inner loop.

In this way, the one or more first predetermined criteria may comprises: the first process being iteratively performed no less than a first predetermined number of iterations; the one or more second image quality metrics changing by less than a first predetermined percentage or amount over a preceding second predetermined number of iterations; and/or a first override indicator being provided.

Similarly, the one or more second predetermined criteria may comprise: the second process being iteratively performed no less than a third predetermined number of iterations; the one or more first image quality metrics changing by less than a second predetermined percentage or amount over a preceding fourth predetermined number of iterations; and/or a second override indicator being provided.

In the context of the present disclosure, a step of obtaining is considered to include the retrieval of pre-stored data (e.g., from an internal memory such as RAM), active retrieval of data from an external database or system and/or passively receiving the data from inter-processor wires.

Thus, referring to Fig. 2, in the second process 200, the step of obtaining the raw or partially processed medical imaging data may, for a first iteration of the second process 220 in the first iteration of the second process, comprise retrieving the raw or partially processed medical imaging data from an external source (e.g., an medical imaging system). In any subsequent iterations of the second process, the step of obtaining the raw or partially processed medical imaging data may comprise retrieving the raw or partially processed medical imaging data from a local or internal memory system.

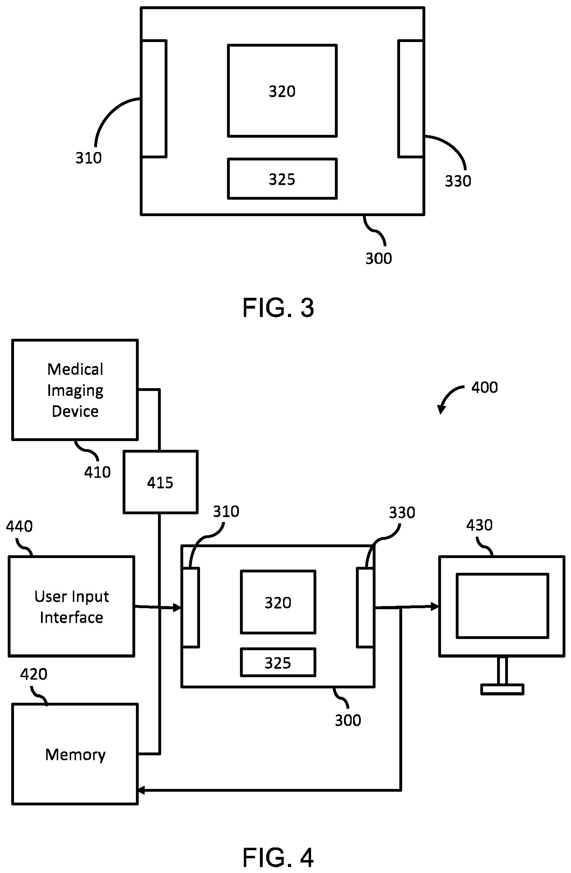

Fig. 3 illustrates a processing system 300 for carrying out a method 200 according to an embodiment.

The processing system 300 comprises an input interface 310. The input interface comprises any circuitry and/or elements required for receiving data from an external device or system for inputting to the processing system 300.

The processing system 300 also comprises a data processor 320. The data processor is configured to carry out the method 200 to produce a set of one or more imaging parameters for use in processing raw or partially-processed medical imaging data to produce medical imaging data (e.g., one or more imaging parameters for a reconstruction process).

The data processor 320 may be configured to perform any obtaining step using the input interface, e.g., when needing to obtain data from an external device.

The data processor 320 can be virtually any custom made or commercially available processor, a central processing unit (CPU), a digital signal processor (DSP), or an auxiliary processor among several processors associated with the processing system XX0. The data processor 320 may be a semiconductor based microprocessor (in the form of a microchip) or a microprocessor.

The processing system may comprise a memory 325. The memory 325 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 325 may incorporate electronic, magnetic, optical, and/or other types of storage media. Note that the memory 325 can have a distributed architecture, where various components are situated remote from one another, but can be accessed by the data processor 320.

The processor 320 may be a hardware device configured for executing software that can be stored in the memory 325. Thus, the memory may store software or a computer program which, when executed by the data processor, causes method 200 to be carried out.

The software in the memory 325 may include one or more separate programs, each of which comprises an ordered listing of executable instructions for implementing logical functions. The software in the memory 325 includes a suitable operating system (O/S) compiler, source code, and one or more applications in accordance with exemplary embodiments. As illustrated, the application comprises numerous functional components for implementing the features and operations of the exemplary embodiments. The application of the processing system may represent various applications, computational units, logic, functional units, processes, operations, virtual entities, and/or modules in accordance with exemplary embodiments, but the application is not meant to be a limitation.

An application may be a source program, executable program (object code), script, or any other entity comprising a set of instructions to be performed. When a source program, then the program is usually translated via a compiler, assembler, interpreter, or the like, which may or may not be included within the memory 325, so as to operate properly in connection with the O/S. Furthermore, the application can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The processing system 300 may comprise an output interface 330. The output interface is an interface configured for passing data to, or controlling, external devices such as a display or user interface. Thus, the output interface comprises any circuitry and/or elements required for transmitting data from the processing system 300 to an external device or system.

The data processor 320 may be configured to use the output interface 330 to output medical imaging data produced by processing the raw or partially-processed medical imaging data using the set of one or more imaging parameters.

In some examples, the data processor is configured to store the output medical imaging data in a database or memory unit, e.g., for later reference and/or retrieval. The storing takes place via the output interface.

In some examples, the data processor 320 is configured to pass the output medical imaging data to a further processing system. The passing takes place via the output interface 330.

In some examples, the data processor 320 is configured to control a user interface to provide a visual representation of the medical imaging data produced by processing the raw or partially-processed medical imaging data using the set of one or more imaging parameters. The controlling takes place via the output interface 330.

The processing system may further comprise internal circuitry for communicatively coupling different elements of the processing system together. The internal circuitry can be, for example but not limited to, one or more buses or other wired or wireless connections, as is known in the art. The internal circuitry may have additional elements, such as controllers, buffers (caches), drivers, repeaters, and receivers, to enable communications. Further, the internal circuitry may include address, control, and/or data connections to enable appropriate communications among the aforementioned components.

In some examples, if the processing system is configured to obtain the partially-processed medical imaging data, the data processor 320 of the processing system 300 may be further configured to obtain (e.g., via the input interface 310) and (partially) process raw medical imaging data to produce the partially-processed medical imaging data.

Fig. 4 illustrates an imaging system 400 according to an embodiment. The imaging system 400 comprises the processing system 300, previously described with reference to Fig. 4.

The imaging system 400 further comprises a medical imaging device 410. The medical imaging device 410 is configured to generate or capture raw medical imaging data for processing by the processing system 300. The raw medical imaging data may be partially processed (e.g., by an intermediate processing system 415 or the processing system 300) to produce-partially processed medical imaging data.

In some examples, the raw and/or partially processed medical imaging data is stored in a storage system 420 or memory of the imaging system, examples of which are well known in the art. The processing system 300 may be configured to retrieve the raw and/or partially processed medical imaging data from the storage system 420.

The processing system 300 may be configured to store the modified set of one or more imaging parameters in a memory or storage unit/system 420. The processing system 300 may be configured to store medical imaging data produced by processing the raw or partially-processed medical imaging data using the (modified) set of one or more imaging parameters.

The imaging system 400 further comprises a user output interface 430, such as a display or monitor. The processing system 300 may be configured to control the user output interface, e.g., via the output interface of the processing system.

In particular, the processing system 300 may be configured to control the user output interface to provide a visual representation of the medical imaging data produced by processing the raw or partially-processed medical imaging data using the set of one or more imaging parameters.

In some examples, the processing system is configured to process the medical imaging data to produce display data, the display data comprising a rendering of the medical imaging data in a format that can be used by the display to provide a visual representation of the medical imaging data.

The imaging system 400 may comprise a user input interface 440. The user input interface may be configured to facilitate control of the medical imaging device 410 via the user input interface. The user input interface 440 may be configured to provide one or more signals or instances of data to the processing system, e.g., one or more override signals (for providing any override indicators); and/or one or more characteristics of the (raw and/or partially-processed) medical imaging data; and/or the property indicator(s).

In any above described embodiments, the one or more imaging parameters may include one or more parameters that are unavailable to an operator (i.e. end-user) of the medical imaging system. In other words, the one or more imaging parameters may include one or more parameters that cannot be adjusted by the operator of the medical imaging device (e.g. at a standard input interface of the medical imaging device). Parameters that are unavailable to the operator are parameters that are not available for adjustment by the operator during standard operation of the medical imaging device, e.g. when it is being used to image a subject. This would not include parameters that could be accessed in a debug mode, an updating mode or any other mode that requires specialized technical knowledge, security or permission to access.

For instance, the one or more imaging parameters may include receive parameters and/or post-processing parameters that cannot be adjusted by an operator of the medical imaging system. There are a wide variety of parameters in a medical imaging system that are not available to a subject (e.g. filtering parameters). The skilled person would be readily capable of identifying a number of other suitable parameters that are not available to the end-user. These approaches significantly increase a flexibility of the proposed approach, and may facilitate improved accuracy in modifying the one or more imaging parameters appropriately (e.g. as they cannot be adjusted or affected by the operator themselves).

Each set of one or more imaging parameters may include at least one or more of receive parameters and post-processing parameters. Receive parameters are the one or more imaging parameters used whilst first processing sampled raw medical imaging data (e.g. an ultrasound image). Post-processing parameters include one or more imaging parameters used after the raw medical imaging data is obtained but not available to the end-user.

Embodiments of the invention are particularly advantageous when the medical imaging data is ultrasound imaging data.

Ultrasound imaging data is particularly sensitive to changes in imaging parameters, and the widespread use of ultrasound imaging data in different environments and for investigating different targets means that imaging parameters designed for providing high quality imaging data in one use-case scenario are not guaranteed to provide high quality imaging data in another use-case scenario.

In any above described embodiments, medical imaging data may comprise, or be data that is processable to derive, one or more 2D medical images and/or one or more 3D medical images. In particular, the processed and/or comparison medical imaging data may comprise the one or more 2D medical images and/or one or more 3D medical images.

It will be understood that disclosed methods are preferably computer-implemented methods. As such, there is also proposed the concept of a computer program comprising code means for implementing any described method when said program is run on a processor or device, such as a computer. Thus, different portions, lines or blocks of code of a computer program according to an embodiment may be executed by a processor or device or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or device or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, particularly a non-tangible storage medium such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

## Claims

1. A computer-implemented method of modifying a set of one or more imaging parameters (192) for a medical imaging process, the computer-implemented method comprising iteratively performing, until one or more first predetermined criteria are met, a first process (210) comprising:
performing, until one or more second predetermined criteria are met, a second process (220) comprising:
obtaining (221) raw or partially-processed medical imaging data;
processing (222) the raw or partially-processed medical imaging data using the set of one or more imaging parameters to produce processed medical imaging data;
determining (223) one or more first image quality metrics (194) by processing the processed medical imaging data using at least one no-reference image quality metric technique; and
modifying (224) the set of one or more imaging parameters responsive to the determined one or more first image quality metrics;
obtaining (211) raw or partially-processed reference medical imaging data (195) used for at least one full-reference image quality metric technique;
processing (212) the raw or partially-processed reference medical imaging data using the set of one or more imaging parameters produced by the second process (220) to produce comparison medical imaging data;
determining (213) one or more second image quality metrics (197) by processing the comparison medical imaging data using the at least one full-reference image quality metric technique; and
modifying (214) the set of one or more imaging parameters responsive to the determined one or more second image quality metrics.

2. The computer-implemented method of claim 1, further comprising, after completing the first process (210), outputting (230) medical imaging data produced by processing the raw or partially-processed medical imaging data using the set of one or more imaging parameters.

3. The computer-implemented method of claim 2, wherein the step of outputting medical imaging data comprises controlling a user interface to provide a visual representation of the medical imaging data produced by processing the raw or partially-processed medical imaging data using the set of one or more imaging parameters.

4. The computer-implemented method of any of claims 1 to 3, wherein the set of one or more imaging parameters includes at least gain and dynamic range.

5. The computer-implemented method of any of claims 1 to 4, wherein the step (213) of determining one or more second image quality metrics comprises:
obtaining a first property indicator that indicates one or more desired properties of medical imaging data produced by processing the raw or partially-processed medical imaging data using the set of one or more imaging parameters;
selecting, from a plurality of potential full-reference image quality metric techniques, one or more full-reference image quality metric techniques responsive to the property indicator; and
generating the one or more second image quality metrics by processing the comparison medical imaging data using the selected one or more full-reference image quality metric techniques.

6. The computer-implemented method of any of claims 1 to 5, wherein the step (214) of modifying the set of one or more imaging parameters responsive to the determined one or more second image quality metrics comprises:
obtaining a second property indicator that indicates one or more desired properties of medical imaging data produced by processing the raw or partially-processed medical imaging data using the set of one or more imaging parameters;
generating a combined image quality metric by weighting each second image quality metric and combining the weighted second image quality metrics; and
modifying the set of one or more imaging parameters responsive to the determined combined image quality metric,
wherein the weighting of each second image quality metric is responsive to the property indicator.

7. The computer-implemented method of any of claims 1 to 6, wherein the obtained raw or partially processed medical imaging data is the same for each iteration of the second process.

8. The computer-implemented method of any of claims 1 to 7, wherein the step (213) of determining one or more second image quality metrics comprises:
obtaining calibration medical imaging data, being processed imaging data representing a known view, material composition, shape, position and/or size of each target; and
processing the comparison medical imaging data and the calibration medical imaging data to produce the one or more second image quality metrics.

9. The computer-implemented method of any of claims 1 to 8, wherein the step of obtaining (211) raw or partially-processed reference medical imaging data comprises, for at least one iteration of the first process:
identifying (211A) a plurality of instances of potential raw or partially-processed reference medical imaging data; and
selecting (211B) one of the plurality of instances of potential raw or partially-processed reference medical imaging data as the obtained raw or partially-processed reference medical imaging data responsive to one or more characteristics of the raw or partially processed medical imaging data.

10. The computer-implemented method of any of claims 1 to 9, wherein the raw or partially processed medical imaging data comprises raw or partially processed ultrasound imaging data.

11. The computer-implemented method of any of claims 1 to 10, wherein the raw or partially processed medical imaging data and the raw or partially-processed reference medical imaging data are medical imaging data at a same location in an image processing pipeline for medical imaging data.

12. The computer-implemented method of any of claims 1 to 11, wherein the one or more first predetermined criteria comprises:
the first process being iteratively performed no less than a first predetermined number of iterations;
the one or more second image quality metrics changing by less than a first predetermined percentage or amount over a preceding second predetermined number of iterations; and/or
a first override indicator being provided.

13. The computer-implemented method of any of claims 1 to 12, wherein the one or more second predetermined criteria comprises:
the second process being iteratively performed no less than a third predetermined number of iterations;
the one or more first image quality metrics changing by less than a second predetermined percentage or amount over a preceding fourth predetermined number of iterations; and/or
a second override indicator being provided.

14. A computer program comprising code means for implementing the method of any one of claims 1 to 13 when said program is run on a processing system.

15. A processing system for modifying a set of one or more imaging parameters (192) for a medical imaging process, the processing system being configured to iteratively perform, until one or more first predetermined criteria are met, a first process comprising:
performing, until one or more second predetermined criteria are met, a second process (220) comprising:
obtaining (221) raw or partially-processed medical imaging data;
processing (222) the raw or partially-processed medical imaging data using the set of one or more imaging parameters to produce processed medical imaging data;
determining (223) one or more first image quality metrics (194) by processing the processed medical imaging data using a no-reference image quality metric technique; and
modifying (224) the set of one or more imaging parameters responsive to the determined one or more first image quality metrics;
obtaining (211) raw or partially-processed reference medical imaging data used for a full-reference image quality metric technique;
processing (212) the raw or partially-processed reference medical imaging data using the set of one or more imaging parameters produced by the second process (220) to produce comparison medical imaging data;
determining (213) one or more second image quality metrics by processing the comparison medical imaging data using the full-reference image quality metric technique; and
modifying (214) the set of one or more imaging parameters responsive to the determined one or more second image quality metrics.

## Patentansprüche

1. Computerimplementiertes Verfahren zum Modifizieren eines Satzes von einem oder mehreren Bildgebungsparametern (192) für einen medizinischen Bildgebungsprozess, wobei das computerimplementierte Verfahren iteratives Durchführen, bis ein oder mehrere vorbestimmte Kriterien erfüllt sind, eines ersten Prozesses (210) umfasst, umfassend:
Durchführen eines zweiten Prozesses (220), bis ein oder mehrere zweite vorbestimmte Kriterien erfüllt sind, umfassend:
Erhalten (221) von rohen oder teilweise verarbeiteten medizinischen Bildgebungsdaten;
Verarbeiten (222) der rohen oder teilweise verarbeiteten medizinischen Bildgebungsdaten unter Verwendung des Satzes von einem oder mehreren Bildgebungsparametern, um verarbeitete medizinische Bildgebungsdaten zu erzeugen;
Bestimmen (223) einer oder mehrerer erster Bildqualitätsmetriken (194) durch Verarbeiten der verarbeiteten medizinischen Bildgebungsdaten unter Verwendung mindestens einer referenzlosen Bildqualitätsmetriktechnik; und
Modifizieren (224) des Satzes von einem oder mehreren Bildgebungsparametern als Reaktion auf die bestimmten eine oder mehreren ersten Bildqualitätsmetriken;
Erhalten (211) von rohen oder teilweise verarbeiteten medizinischen Bildgebungsreferenzdaten (195), die für mindestens eine vollständige Referenzbildqualitätsmetriktechnik verwendet werden;
Verarbeiten (212) der rohen oder teilweise verarbeiteten medizinischen Bildgebungsreferenzdaten unter Verwendung des Satzes von einem oder mehreren Bildgebungsparametern, die durch den zweiten Prozess (220) erzeugt wurden, um medizinische Bildgebungsvergleichsdaten zu erzeugen;
Bestimmen (213) einer oder mehrerer zweiter Bildqualitätsmetriken (197) durch Verarbeiten der medizinischen Bildgebungsvergleichsdaten unter Verwendung der mindestens einen vollständigen Referenzbildqualitätsmetriktechnik; und
Modifizieren (214) des Satzes von einem oder mehreren Bildgebungsparametern als Reaktion auf die bestimmten zweiten Bildqualitätsmetriken.

2. Computerimplementiertes Verfahren nach Anspruch 1, das weiter nach Abschluss des ersten Prozesses (210) Ausgeben (230) von medizinischen Bildgebungsdaten umfasst, die durch Verarbeiten der rohen oder teilweise verarbeiteten medizinischen Bildgebungsdaten unter Verwendung des Satzes von einem oder mehreren Bildgebungsparametern erzeugt wurden.

3. Computerimplementiertes Verfahren nach Anspruch 2, wobei der Schritt zum Ausgeben medizinischer Bildgebungsdaten Steuern einer Benutzeroberfläche umfasst, um eine visuelle Darstellung der medizinischen Bildgebungsdaten bereitzustellen, die durch Verarbeiten der rohen oder teilweise verarbeiteten medizinischen Bildgebungsdaten unter Verwendung des Satzes von einem oder mehreren Bildgebungsparametern erzeugt wurden.

4. Computerimplementiertes Verfahren nach einem der Ansprüche 1 bis 3, wobei der Satz von einem oder mehreren Bildgebungsparametern mindestens Verstärkung und Dynamikbereich beinhaltet.

5. Computerimplementiertes Verfahren nach einem der Ansprüche 1 bis 4, wobei der Schritt (213) zum Bestimmen einer oder mehrerer zweiter Bildqualitätsmetriken umfasst:
Erhalten eines ersten Eigenschaftsindikators, der eine oder mehrere gewünschte Eigenschaften von medizinischen Bildgebungsdaten indiziert, die durch Verarbeiten der rohen oder teilweise verarbeiteten medizinischen Bildgebungsdaten unter Verwendung des Satzes von einem oder mehreren Bildgebungsparametern erzeugt wurden;
Auswählen aus einer Vielzahl von potenziellen vollständigen Referenzbildqualitätsmetriktechniken einer oder mehrerer vollständiger Referenzbildqualitätsmetriktechniken als Reaktion auf den Eigenschaftsindikator; und
Generieren der einen oder mehreren zweiten Bildqualitätsmetriken durch Verarbeiten der medizinischen Bildgebungsvergleichsdaten unter Verwendung der ausgewählten einen oder mehreren vollständigen Referenzbildqualitätsmetriktechniken.

6. Computerimplementiertes Verfahren nach einem der Ansprüche 1 bis 5, wobei der Schritt (214) zum Modifizieren des Satzes von einem oder mehreren Bildgebungsparametern als Reaktion auf die bestimmten eine oder mehreren zweiten Bildqualitätsmetriken umfasst:
Erhalten eines zweiten Eigenschaftsindikators, der eine oder mehrere gewünschte Eigenschaften von medizinischen Bildgebungsdaten indiziert, die durch Verarbeiten der rohen oder teilweise verarbeiteten medizinischen Bildgebungsdaten unter Verwendung des Satzes von einem oder mehreren Bildgebungsparametern erzeugt wurden;
Generieren einer kombinierten Bildqualitätsmetrik durch Gewichten jeder zweiten Bildqualitätsmetrik und Kombinieren der gewichteten zweiten Bildqualitätsmetriken; und
Modifizieren des Satzes von einem oder mehreren Bildgebungsparametern als Reaktion auf die bestimmte kombinierte Bildqualitätsmetrik,
wobei das Gewichten jeder zweiten Bildqualitätsmetrik als Reaktion auf den Eigenschaftsindikator ist.

7. Computerimplementiertes Verfahren nach einem der Ansprüche 1 bis 6, wobei die erhaltenen rohen oder teilweise verarbeiteten medizinischen Bildgebungsdaten für jede Iteration des zweiten Prozesses gleich sind.

8. Computerimplementiertes Verfahren nach einem der Ansprüche 1 bis 7, wobei der Schritt (213) zum Bestimmen einer oder mehrerer zweiter Bildqualitätsmetriken umfasst:
Erhalten von medizinischen Bildgebungskalibrierungsdaten, die verarbeitete Bildgebungsdaten sind, die eine bekannte Ansicht, Materialzusammensetzung, Form, Position und/oder Größe jedes Ziels darstellen; und
Verarbeiten der medizinischen Bildgebungsvergleichsdaten und der medizinischen Bildgebungskalibrierungsdaten, um die eine oder mehreren zweiten Bildqualitätsmetriken zu erzeugen.

9. Computerimplementiertes Verfahren nach einem der Ansprüche 1 bis 8, wobei der Schritt zum Erhalten (211) von rohen oder teilweise verarbeiteten medizinischen Bildgebungsreferenzdaten für mindestens eine Iteration des ersten Prozesses umfasst:
Identifizieren (211A) einer Vielzahl von Fällen potenzieller roher oder teilweise verarbeiteter medizinischer Bildgebungsreferenzdaten; und
Auswählen (211B) eines der Vielzahl von Fällen potenzieller roher oder teilweise verarbeiteter medizinische Bildgebungsreferenzdaten als die erhaltenen rohen oder teilweise verarbeiteten medizinischen Bildgebungsreferenzdaten, als Reaktion auf eine oder mehrere Eigenschaften der rohen oder teilweise verarbeiteten medizinischen Bildgebungsdaten.

10. Computerimplementiertes Verfahren nach einem der Ansprüche 1 bis 9, wobei die rohen oder teilweise verarbeiteten medizinischen Bildgebungsdaten rohe oder teilweise verarbeitete Ultraschallbildgebungsdaten umfassen.

11. Computerimplementiertes Verfahren nach einem der Ansprüche 1 bis 10, wobei die rohen oder teilweise verarbeiteten medizinischen Bildgebungsdaten und die rohen oder teilweise verarbeiteten medizinischen Bildgebungsreferenzdaten medizinische Bildgebungsdaten an einer gleichen Stelle in einer Bildverarbeitungspipeline für medizinische Bildgebungsdaten sind.

12. Computerimplementiertes Verfahren nach einem der Ansprüche 1 bis 11, wobei das eine oder die mehreren ersten vorbestimmten Kriterien umfassen:
dass der erste Prozess nicht weniger als eine vorbestimmte Anzahl an Iterationen iterativ durchgeführt wird;
dass sich die eine oder mehreren zweiten Bildqualitätsmetriken um weniger als einen ersten vorbestimmten Prozentsatz oder Betrag gegenüber einer vorhergehenden zweiten vorbestimmten Anzahl an Iterationen ändern; und/oder
dass ein erster Überschreibungsindikator bereitgestellt wird.

13. Computerimplementiertes Verfahren nach einem der Ansprüche 1 bis 12, wobei das eine oder die mehreren zweiten vorbestimmten Kriterien umfassen:
dass der zweite Prozess nicht weniger als ein Drittel vorbestimmter Anzahl an Iterationen durchgeführt wird;
dass sich die eine oder mehreren ersten Bildqualitätsmetriken um weniger als einen zweiten vorbestimmten Prozentsatz oder Betrag gegenüber einer vorhergehenden vierten vorbestimmten Anzahl an Iterationen ändern; und/oder
dass ein zweiter Überschreibungsindikator bereitgestellt wird.

14. Computerprogramm, das Codemittel zum Implementieren des Verfahrens nach einem der Ansprüche 1 bis 13 umfasst, wenn das Programm in einem Verarbeitungssystem läuft.

15. Verarbeitungssystem zum Modifizieren eines Satzes von einem oder mehreren Bildgebungsparametern (192) für einen medizinischen Bildgebungsprozess, wobei das Verarbeitungssystem konfiguriert ist, iterativ einen ersten Prozess durchzuführen, bis ein oder mehrere vorbestimmte Kriterien erfüllt sind, der erste Proyess umfassend:
Durchführen eines zweiten Prozesses (220), bis ein oder mehrere zweite vorbestimmte Kriterien erfüllt sind, umfassend:
Erhalten (221) von rohen oder teilweise verarbeiteten medizinischen Bildgebungsdaten;
Verarbeiten (222) der rohen oder teilweise verarbeiteten medizinischen Bildgebungsdaten unter Verwendung des Satzes von einem oder mehreren Bildgebungsparametern, um verarbeitete medizinische Bildgebungsdaten zu erzeugen;
Bestimmen (223) einer oder mehrerer erster Bildqualitätsmetriken (194) durch Verarbeiten der verarbeiteten medizinischen Bildgebungsdaten unter Verwendung einer referenzlosen Bildqualitätsmetriktechnik; und
Modifizieren (224) des Satzes von einem oder mehreren Bildgebungsparametern als Reaktion auf die bestimmten eine oder mehreren ersten Bildqualitätsmetriken;
Erhalten (211) von rohen oder teilweise verarbeiteten medizinischen Bildgebungsreferenzdaten, die für eine vollständige Referenzbildqualitätsmetriktechnik verwendet werden;
Verarbeiten (212) der rohen oder teilweise verarbeiteten medizinischen Bildgebungsreferenzdaten unter Verwendung des Satzes von einem oder mehreren Bildgebungsparametern, die durch den zweiten Prozess (220) erzeugt wurden, um medizinische Bildgebungsvergleichsdaten zu erzeugen;
Bestimmen (213) einer oder mehrerer zweiter Bildqualitätsmetriken durch Verarbeiten der medizinischen Bildgebungsvergleichsdaten unter Verwendung der vollständigen Referenzbildqualitätsmetriktechnik; und
Modifizieren (214) des Satzes von einem oder mehreren Bildgebungsparametern als Reaktion auf die bestimmten zweiten Bildqualitätsmetriken.

## Revendications

1. Procédé mis en œuvre par ordinateur consistant à modifier un ensemble d'un ou plusieurs paramètres d'imagerie (192) pour un processus d'imagerie médicale, le procédé mis en œuvre par ordinateur comprenant l'exécution itérative, jusqu'à ce qu'un ou plusieurs premiers critères prédéterminés soient satisfaits, d'un premier processus (210) comprenant :
l'exécution, jusqu'à ce qu'un ou plusieurs seconds critères prédéterminés soient satisfaits, d'un second processus (220) comprenant :
l'obtention (221) de données d'imagerie médicale brutes ou partiellement traitées ;
le traitement (222) des données d'imagerie médicale brutes ou partiellement traitées à l'aide de l'ensemble d'un ou plusieurs paramètres d'imagerie pour produire des données d'imagerie médicale traitées ;
la détermination (223) d'une ou plusieurs premières mesures de qualité d'image (194) en traitant les données d'imagerie médicale traitées à l'aide d'au moins une technique de mesure de qualité d'image sans référence ; et
la modification (224) de l'ensemble d'un ou plusieurs paramètres d'imagerie répondant aux une ou plusieurs premières mesures de qualité d'image déterminées ;
l'obtention (211) de données d'imagerie médicale de référence brutes ou partiellement traitées (195) utilisées pour au moins une technique de mesure de qualité d'image de référence complète ;
le traitement (212) des données d'imagerie médicale de référence brutes ou partiellement traitées à l'aide de l'ensemble d'un ou plusieurs paramètres d'imagerie produits par le second processus (220) pour produire des données d'imagerie médicale de comparaison ;
la détermination (213) d'une ou plusieurs secondes mesures de qualité d'image (197) en traitant les données d'imagerie médicale de comparaison à l'aide de la au moins une technique de mesure de qualité d'image de référence complète ; et
la modification (214) de l'ensemble d'un ou plusieurs paramètres d'imagerie répondant aux une ou plusieurs secondes mesures de qualité d'image déterminées.

2. Procédé mis en œuvre par ordinateur selon la revendication 1, comprenant en outre, après avoir terminé le premier processus (210), l'émission (230) de données d'imagerie médicale produites en traitant les données d'imagerie médicale brutes ou partiellement traitées à l'aide de l'ensemble d'un ou plusieurs paramètres d'imagerie.

3. Procédé mis en œuvre par ordinateur selon la revendication 2, dans lequel l'étape d'émission des données d'imagerie médicale comprend la commande d'une interface utilisateur pour fournir une représentation visuelle des données d'imagerie médicale produites par le traitement des données d'imagerie médicale brutes ou partiellement traitées à l'aide de l'ensemble d'un ou plusieurs paramètres d'imagerie.

4. Procédé mis en œuvre par ordinateur selon l'une quelconque des revendications 1 à 3, dans lequel l'ensemble d'un ou plusieurs paramètres d'imagerie inclut au moins le gain et la plage dynamique.

5. Procédé mis en œuvre par ordinateur selon l'une quelconque des revendications 1 à 4, dans lequel l'étape (213) de détermination d'une ou plusieurs secondes mesures de qualité d'image comprend :
l'obtention d'un premier indicateur de propriété qui indique une ou plusieurs propriétés souhaitées des données d'imagerie médicale produites par le traitement des données d'imagerie médicale brutes ou partiellement traitées à l'aide de l'ensemble d'un ou plusieurs paramètres d'imagerie ;
la sélection, parmi une pluralité de techniques potentielles de mesure de qualité d'image de référence complète, d'une ou plusieurs techniques de mesure de qualité d'image de référence complète répondant à l'indicateur de propriété ; et
la génération des une ou plusieurs secondes mesures de qualité d'image en traitant les données d'imagerie médicale de comparaison à l'aide des une ou plusieurs techniques de mesure de qualité d'image de référence complète sélectionnées.

6. Procédé mis en œuvre par ordinateur selon l'une quelconque des revendications 1 à 5, dans lequel l'étape (214) de modification de l'ensemble d'un ou plusieurs paramètres d'imagerie répondant aux une ou plusieurs secondes mesures de qualité d'image déterminées comprend :
l'obtention d'un second indicateur de propriété qui indique une ou plusieurs propriétés souhaitées des données d'imagerie médicale produites par le traitement des données d'imagerie médicale brutes ou partiellement traitées à l'aide de l'ensemble d'un ou plusieurs paramètres d'imagerie ;
la génération d'une mesure de qualité d'image combinée en pondérant chaque seconde mesure de qualité d'image et en combinant les secondes mesures de qualité d'image pondérées ; et
la modification de l'ensemble d'un ou plusieurs paramètres d'imagerie répondant à la mesure de qualité d'image combinée déterminée,
dans lequel la pondération de chaque seconde mesure de qualité d'image répond à l'indicateur de propriété.

7. Procédé mis en œuvre par ordinateur selon l'une quelconque des revendications 1 à 6, dans lequel les données d'imagerie médicale brutes ou partiellement traitées obtenues sont les mêmes pour chaque itération du second processus.

8. Procédé mis en œuvre par ordinateur selon l'une quelconque des revendications 1 à 7, dans lequel l'étape (213) de détermination d'une ou plusieurs secondes mesures de qualité d'image comprend :
l'obtention de données d'imagerie médicale d'étalonnage, qui sont des données d'imagerie traitées représentant une vue, une composition matérielle, une forme, une position et/ou une taille connues de chaque cible ; et
le traitement des données d'imagerie médicale de comparaison et des données d'imagerie médicale d'étalonnage pour produire les une ou plusieurs secondes mesures de qualité d'image.

9. Procédé mis en œuvre par ordinateur selon l'une quelconque des revendications 1 à 8, dans lequel l'étape d'obtention (211) de données d'imagerie médicale de référence brutes ou partiellement traitées comprend, pour au moins une itération du premier processus :
l'identification (211A) d'une pluralité d'instances de données d'imagerie médicale de référence brutes ou partiellement traitées potentielles ; et
la sélection (211B) de l'une de la pluralité d'instances de données d'imagerie médicale de référence brutes ou partiellement traitées potentielles comme données d'imagerie médicale de référence brutes ou partiellement traitées obtenues répondant à une ou plusieurs caractéristiques des données d'imagerie médicale brutes ou partiellement traitées.

10. Procédé mis en œuvre par ordinateur selon l'une quelconque des revendications 1 à 9, dans lequel les données d'imagerie médicale brutes ou partiellement traitées comprennent des données d'imagerie par ultrasons brutes ou partiellement traitées.

11. Procédé mis en œuvre par ordinateur selon l'une quelconque des revendications 1 à 10, dans lequel les données d'imagerie médicale brutes ou partiellement traitées et les données d'imagerie médicale de référence brutes ou partiellement traitées sont des données d'imagerie médicale situées au même emplacement dans un pipeline de traitement d'images pour les données d'imagerie médicale.

12. Procédé mis en œuvre par ordinateur selon l'une quelconque des revendications 1 à 11, dans lequel les un ou plusieurs premiers critères prédéterminés comprennent :
le premier processus est exécuté de manière itérative pour au moins un premier nombre prédéterminé d'itérations ;
les une ou plusieurs secondes mesures de qualité d'image changent de moins d'un premier pourcentage ou d'une première quantité prédéterminés sur un deuxième nombre d'itérations prédéterminé précédent ; et/ou
un premier indicateur de priorité est fourni.

13. Procédé mis en œuvre par ordinateur selon l'une quelconque des revendications 1 à 12, dans lequel les un ou plusieurs seconds critères prédéterminés comprennent :
le second processus est exécuté de manière itérative pour au moins un troisième nombre prédéterminé d'itérations ;
les une ou plusieurs premières mesures de qualité d'image changent de moins d'un second pourcentage ou d'une seconde quantité prédéterminés sur un quatrième nombre prédéterminé d'itérations précédent ; et/ou
un second indicateur de priorité est fourni.

14. Programme informatique comprenant des moyens de code pour mettre en œuvre le procédé selon l'une quelconque des revendications 1 à 13 lorsque ledit programme est exécuté sur un système de traitement.

15. Système de traitement pour modifier un ensemble d'un ou plusieurs paramètres d'imagerie (192) pour un processus d'imagerie médicale, le système de traitement étant configuré pour exécuter de manière itérative, jusqu'à ce qu'un ou plusieurs premiers critères prédéterminés soient satisfaits, un premier processus comprenant :
l'exécution, jusqu'à ce qu'un ou plusieurs seconds critères prédéterminés soient satisfaits, d'un second processus (220) comprenant :
l'obtention (221) de données d'imagerie médicale brutes ou partiellement traitées ;
le traitement (222) des données d'imagerie médicale brutes ou partiellement traitées à l'aide de l'ensemble d'un ou plusieurs paramètres d'imagerie pour produire des données d'imagerie médicale traitées ;
la détermination (223) d'une ou plusieurs premières mesures de qualité d'image (194) en traitant les données d'imagerie médicale traitées à l'aide d'une technique de mesure de qualité d'image sans référence ; et
la modification (224) de l'ensemble d'un ou plusieurs paramètres d'imagerie répondant aux une ou plusieurs premières mesures de qualité d'image déterminées ;
l'obtention (211) de données d'imagerie médicale de référence brutes ou partiellement traitées utilisées pour une technique de mesure de qualité d'image de référence complète ;
le traitement (212) des données d'imagerie médicale de référence brutes ou partiellement traitées à l'aide de l'ensemble d'un ou plusieurs paramètres d'imagerie produits par le second processus (220) pour produire des données d'imagerie médicale de comparaison ;
la détermination (213) d'une ou plusieurs secondes mesures de qualité d'image en traitant les données d'imagerie médicale de comparaison à l'aide de la technique de mesure de qualité d'image de référence complète ; et
la modification (214) de l'ensemble d'un ou plusieurs paramètres d'imagerie répondant aux une ou plusieurs secondes mesures de qualité d'image déterminées.
